(19) Europäisches Patentamt
European Patent Office
Office européen des brevets

(11) Numéro de publication: **0 337 866 B1**

(12) # FASCICULE DE BREVET EUROPEEN

(45) Date de publication de fascicule du brevet: **01.09.93**

(51) Int. Cl.5: **A61K 31/37**

(21) Numéro de dépôt: **89400976.0**

(22) Date de dépôt: **10.04.89**

(54) **5-Méthoxy-psoralène comme anti-dépresseur.**

(30) Priorité: **14.04.88 FR 8804954**

(43) Date de publication de la demande:
**18.10.89 Bulletin 89/42**

(45) Mention de la délivrance du brevet:
**01.09.93 Bulletin 93/35**

(84) Etats contractants désignés:
**AT BE CH DE ES FR GB GR IT LI LU NL SE**

(56) Documents cités:

**PSYCHOPHARMACOLOGY, vol. 95, no. 3, 1988, Springer-Verlag; E. SOUETRE et al., pp. 430-431&NUM;**

**J. INVEST DERMATOL., vol. 82, no. 2, 1987; E. SOUETRE et al., pp. 152-155&NUM;**

**J. AM. ACAD. CHILD. ADOLESC. PSYCHIATRY, vol. 26, no. 3, 1987, American Academy of Child and Adolescent Psychiatry; E. CAVALLO et al., pp. 395-399&NUM;**

**AUSTRALIAN AND NEW ZEALAND JOURNAL OF PSYCHIATRY, vol. 20, no. 3, 1986; I.M. McINTYRE et al., pp. 381-383&NUM;**

**BIOL. PSYCHIATRY, vol. 21, 1986; R.L. SACK et al., pp. 406-410&NUM;**

**DISS. PHARM. PHARMACOL., vol. 23, no. 6, 1971; K.S. ROUSSINOV, pp. 634-635&NUM;**

(73) Titulaire: **Goupil, Jean-Jacques**
**23 Ouai Le Gallo**
**Boulogne (Hauts de Seine)(FR)**

(72) Inventeur: **Goupil, Jean-Jacques**
**23 Ouai Le Gallo**
**Boulogne (Hauts de Seine)(FR)**

(74) Mandataire: **Petit, Hélène**
**Cabinet Hélène Petit 94, Avenue Kléber**
**F-75116 Paris (FR)**

**EP 0 337 866 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet un nouveau médicament anti-dépresseur, efficace dans la dépression endogène, caractérisé en ce qu'il contient à titre de substance active du 5-méthoxy-psoralène de formule $C_{12}H_8O_4$.

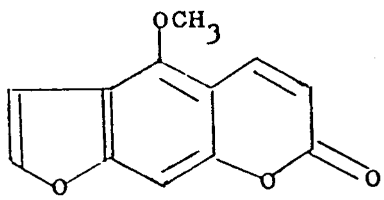

On sait que le 5-méthoxy-psoralène a une activité dans le traitement du psoriasis lorsque son administration est associée à une irradiation aux ultraviolets A.

Les études de toxicité du 5-méthoxy-psoralène, effectuées sur les souris et les rats mâles, ont montré que cette molécule pouvait être facilement administrée dans de larges doses du fait d'une faible toxicité. Ces expérimentations sont rappelées ci-après.

Les expérimentations ont été effectuées sur des souris et des rats mâles, exempts d'organismes pathogènes spécifiques et soumis à une diète hydrique préalable de 18 heures. Tous les animaux ont été gardés en observation pendant 14 jours après l'administration orale unique afin de détecter une éventuelle toxicité retardée. La dose léthale 50 et ses limites de confiance ont été calculées selon la méthode graphique de J.T. Litchfield et F. Wilcoxon.

Dans ces essais, l'administration se faisait par voie orale, l'agent dispersif était une solution aqueuse de carboxy-méthyl-cellulose à 2p.100 additionnée de 0,5 p.100 de Tween® 80. Chez les souris mâles on a trouvé une DL 50 en 14 jours de 875 mg/kg pour le 8-méthoxy-psoralène et de 8100 mg/kg pour le 5-méthoxy-psoralène. Chez les rats mâles, on a trouvé une DL 50 en 14 jours de 4400 mg/kg pour le 8-méthoxy-psoralène et supérieure à 30.000 mg/kg pour le 5-méthoxy-psoralène.

On a également étudié la toxicité générale à long terme du 5-méthoxy-psoralène dans des traitements de lapins par voie gastrique et par application dermique. Les résultats sont les suivants.

Pour une administrationi par voie gastrique pendant 42 jours consécutifs de 5-méthoxy-psoralène à la dose quotidienne de 300 mg/kg, l'étude toxicologique (état géneral, examen de la peau et du fond de l'oeil, examen hématologique, de chimie clinique, et anatomopathologique) démontre la bonne tolérance du nouveau médicament et ne fournit aucun élément susceptible de limiter son utilisation contrôlée en clinique humaine.

Le 5-méthoxy-psoralène peut être obtenu par extraction de l'essence de bergamote naturelle ou par des procédés de synthèse.

Dans le cadre de la présente invention, on sait que la dépression endogène s'accompagne d'un certain nombre d'anomalies des rythmes circadiens qui font évoquer une perturbation dans l'entraînement des horloges biologiques par les repères temporels de l'environnement. Des troubles de la synchronisation sont démontrés par la réduction de l'amplitude et l'instabilité des phases des rythmes biologiques (veille/sommeil, mélatonine, cortisol....).

Il a par ailleurs été démontré que l'administration de 5-méthoxy-psoralène à des sujets parfaitement sains, provoquait une augmentation de la secrétion de la mélatonine, sans avoir par ailleurs aucun effet sur les autres hormones endocriniennes.

Selon la présente invention, il a été mis en évidence que le 5-méthoxy-psoralène, administré à divers sujets psychiatriques tels que des dépressifs, des schizophrènes et des déments, provoquait une élévation significative des taux plasmatiques de mélatonine. En ce qui concerne les sujets dépressifs, on a pu montrer que l'administration de 5-méthoxy-psoralène entraînait très rapidement une amélioration très significative de l'état dépressif.

Etude de l'administration de 5-méthoxy-psoralène dans le cadre de pathologies psychiatriques variées.

L'expérimentation a été effectuée sur six groupes de sujets.

Les groupes contrôles 1, 2, 3 comprenaient respectivement

groupe 1, 13 sujets sains âgés (7 femmes, 6 hommes)

groupe 2, 13 sujets sains âge moyen (6 femmes, 7 hommes)

groupe 3, 13 jeunes adultes sains (6 femmes, 7 hommes).

Ces sujets avaient été adressés soit pour des problèmes sociaux ou des problèmes de nervosité mineure, mais sans trouble psychiatrique majeur, ni maladie organique.

La plupart des sujets âgés étaient dans l'attente d'un placement en maison de retraite.

Le groupe 4 comprenait 13 patients (7 femmes, 6 hommes) présentant tous les critères de la démence de la classification du DSM III. Ces patients âgés ne présentaient pas de troubles majeurs, cardiaques, hépatiques, rénaux, endocriniens ou osseux, ni de pathologie évolutive.

Le groupe 5 comprenait 13 sujets en phase dépressive aigue (8 femmes, 5 hommes) rassemblant tous les critères d'un désordre dépressif majeur au sens du DSM III.

Le groupe 6 comprenait 12 sujets schizophrènes (6 femmes, 7 hommes) suivant les critères du DSM III.

Le diagnostic était établi selon un interrogatoire structuré pour les désordres affectifs, la schizophrénie et la démence, interrogatoire mené pour chaque patient par deux psychiatres différents.

Tout sujet présentant une histoire de maladie grave concommittante tel HTA, diabète ou autre pathologie endocrinienne, avec des résultats cliniques ou biologiques perturbés était exclu du protocole.

Les patients ne reçurent aucun traitement médicamenteux les 4 jours précédant le test en dehors de 5 mg de nitrazépam ou 0,25 mg de trazolam le soir.

Tous les sujets participaient volontairement à l'étude et étaient informés de son but.

Une seule prise orale de 40 mg de 5 méthoxy-psoralène ou 5 MOP était donnée à 20 h à tous les sujets.

Pour éviter tout effet lié aux propriétés photosensibilatrices du psoralène (4) les patients étaient maintenus sous une faible lumière (sans UV) ou dans l'obscurité complète pendant au moins 9 heures après la prise de 5 MOP (période correspondant à la période de sommeil).

Les échantillons sanguins étaient prélevés à 20 h, avant la prise de 5 MOP puis à 23 h grâce à un cathéter placé à demeure sur l'avant-bras au moins 20 minutes avant le prélèvement du premier échantillon.

Ces échantillons étaient immédiatement centrifugés à 4° C et refroidis à -20°C jusqu'au dosage.

Les taux plasmatiques étaient évalués par radio-immunologie après extraction au diethyléther en utilisant la méthode de Treifenaueret Andres.

La fiabilité du dosage de la mélatonine était vérifiée par l'étude des réactions croisées avec d'autres indolamines.

Aucune réaction croisée n'a été décelée entre le dosage de la mélatonine et celui du 5 MOP aux doses utilisées.

Les réactions à l'intérieur d'un même dosage étaient inférieures à 6 % pour une concentration de 0,58 nmol/l. La sensibilité du dosage était de 0,0228 nmol/l.

Les taux de mélatonine étaient établis et comparés dans chaque groupe au moyen de t-tests appariés et entre les groupes utilisant des t-tests non appariés.

Comme l'âge pouvait influencer la réponse endocrinienne nous avons seulement comparé les groupes de moyenne d'âge semblable.

Le "Δ mélatonine" était défini comme étant la différence entre les taux de mélatonine plasmatiques après 5 MOP et ceux de la ligne de base, et p mesure la significativité de la différence entre 2 valeurs.

Les résultats de ces expérimentations ont été les suivants.

Aucun des patients n'a présenté d'effets secondaires après l'absorption du 5 MOP.

Les résultats par groupes sont donnés dans les tableaux 1 et 2.

Les taux de mélatonine étaient fortement augmentés après la prise de 5 MOP dans les groupes de contrôle ($t = 4,59$ $p < 0,01$) ($t = 4,18$ $p < 0,001$) ($t = 3,92$ $p < 0,001$) chez les déments ($t = 3,34$ $p < 0,01$) chez les dépressifs ($t = 2,29$ $p < 0,05$) et les schizophrènes ($t = 8,31$ $p < 0,0001$).

La moyenne "Δ mélatonine" était significativement plus basse chez les déments que chez les sujets âgés du groupe de contrôle ($r = 2,4$ $p < 0,03$).

Ce "Δ mélatonine" était légèrement mais pas significativement réduit chez les dépressifs comparé au groupe contrôle ($r = 0,59$ $p > 0,05$).

Les tableaux 1 et 2 sont présentés dans les pages suivantes.

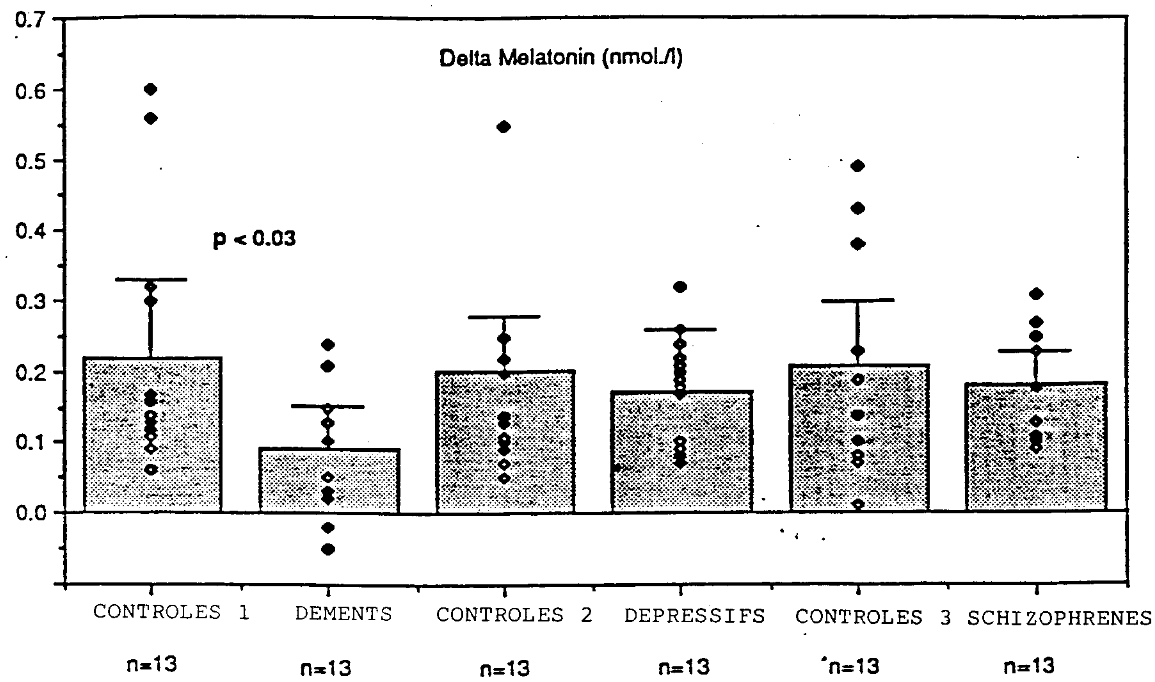


Tableau N° 2

Résultats du " Δ mélatonine" avant et après traitement

Courbes individuelles et moyennes (+ SEM) des " Δ mélatonine" dans les différents groupes de patients.

" Δ mélatonine" = différence entre les taux de mélatonine de la ligne de base et après 5 MOP.

| TABLE 4 | n | âge | Mélatonine (8 p.m.) | Mélatonine (11 p.m.) | Delta Mélatonine |
|---|---|---|---|---|---|
| CONTROLES 1 | 13 | 73.31 ± 5.72 | 0.19 ± 0,05 | 0,42 ± 0,12 | 0,22 ± 0,11 |
| DEMENTS | 13 | 77,92 ± 3,54 | 0,21 ± 0,06 | 0,30 ± 0,08 | 0,09 ± 0,06 |
| valeurs t et p | | 1,56/0,133 | 0,55/0,589 | 1,88/0,073 | 2,4/0,025 |
| | | | | | |
| CONTROLES 2 | 13 | 52,15 ± 7,55 | 0,16 ± 0,04 | 0,36 ± 0,09 | 0,20 ± 0,08 |
| DEPRIMES | 13 | 55,46 ± 9,49 | 0,22 ± 0,06 | O,39 ± 0,15 | 0,17 ± 0,09 |
| valeurs t et p | | 0,41/0,558 | 1,79/O,112 | O,38/0,681 | 0,59/0,562 |
| | | | | | |
| CONTROLES 3 | 13 | 33,46 ± 5,14 | 0,16 ± 0,05 | 0,36 ±0,11 | 0,21 ± 0,09 |
| SCHIZOPHRENES | 13 | 35,20 ± 6,54 | 0,21 ± 0,09 | 0,48 ± 0,09 | 0,15 ± 0,05 |
| VALEURS T ET P | | 0,38/0,708 | 1,35/0,189 | 0,43/0,670 | 0,58/0,567 |

Tableau N° 1

Moyennes et SEM d'âge, taux de ligne de base de la mélatonine plasmatique Taux de mélatonine plasmatique post-5 MOP et le "Δ mélatonine" sont indiqués pour chaque groupe.

En conclusion cette expérimentation a montré une élévation significative des taux plasmatiques de mélatonine chez des sujets psychiatriques après l'administration de 5-méthoxy-psoralène.

Etude clinique de l'effet anti-dépresseur du 5-méthoxy-psoralène

L'expérimentation des effets cliniques du 5-méthoxy-psoralène chez des patients dépressifs a été conduite sur 24 sujets physiquement sains, (11 hommes et 13 femmes), souffrant d'un désordre dépressif majeur.

Tout sujet qui présentait une maladie psychiatrique concommitente telle une démence, une schizophrénie ou une maladie somatique majeure était exclu de l'expérimentation. Les patients choisis atteints d'un désordre dépressif majeur selon le DSM III étaient restés sans traitement pendant les quatre jours avant l'essai, à l'exception de 5 mg de nitrazépam le soir.

Les patients ont été séparés en deux groupes. Le premier groupe a fait l'objet d'un traitement par prise orale quotidienne de 40 mg de 5-méthoxy-psoralène administré à 21 heures pendant une semaine. le deuxième groupe a fait l'objet d'un traitement par prise orale d'un placebo administré en double aveugle dans des conditions identiques.

Pour prévenir tout effet parallèle en liaison avec les propriétés photosensibilisantes du 5-méthoxy-psoralène, les patients ne furent exposés à aucun rayon UV dans les neuf heures qui suivaient la prise de 5 MOP (ce qui correspondait avec leur période de sommeil).

Après une semaine de traitement par le 5-méthoxy-psoralène ou le placebo, les résultats cliniques ont été établis dans chaque groupe de patients et comparés en utilisant le Wilcoxon Test (W) à l'intérieur d'un groupe et le Manswhitmey Test (U) entre les deux groupes. Les résultats ont montré qu'aucun des patients traités par le 5-méthoxy-psoralène n'a présenté d'effets secondaires au traitement.

La ligne de base des cotations HDRS ne différait pas d'un groupe à l'autre.

Les patients qui avaient reçu le placebo ne montrèrent que peu ou pas du tout d'amélioration, alors que les patients qui avaient reçu le 5-méthoxy-psoralène montrèrent une amélioration significative de leur état dépressif.

Les résultats cliniques utilisant les 17 items de l'HDRS sont rassemblés dans le tableau No. 3.

| | N | Sexe ratio | Moyenne d'âge | HDRS JO | HDRS J7 | Valeurs W et p |
|---|---|---|---|---|---|---|
| DESORDRE DEPRESSIF MAJEUR | | | | | | |
| PLACEBO | 12 | 6F/6M | 52,04 ± 12,11 | 22,33 ± 5,03 | 21,16 ± 5,02 | 15p 0,05 |
| 5 MOP | 12 | 7F/5M | 53,83 ± 13,16 | 21,92 ± 8,21 | 16,67 ± 7,99 | 3,5p 0,01 |
| valeurs U et P | | | 64/p 0,05 | 61/p 0,05 | 37/p 0,05 | |

Tableau 3

Résultats cliniques utilisant les 17 items de l'HDRS

Il apparaît que le 5-méthoxy-psoralène présente des propriétés particulièrement remarquables dans l'amélioration extrêmement rapide de l'état dépressif, ceci pour une dose journalière relativement faible.

Par comparaison avec des traitements classiques des états dépressifs par les tricyclides, il apparaît que l'efficacité du nouveau médicament à base de 5-méthoxy-psoralène est particulièrement élevée puisque l'on obtient les mêmes résultats en termes d'amélioration pour un traitement d'une durée d'une semaine avec le nouveau médicament au lieu de trois semaines avec les tricyclides.

Les expérimentations ont montré que l'administration du nouveau médicament devait impérativement avoir lieu le soir au coucher, pour obtenir l'effet anti-dépresseur qui vient d'être décrit.

Dans l'administration par voie orale les doses journalières de 5-méthoxy-psoralène varient entre 0,2 mg et 1 mg/kg de poids corporel du patient, et se situe généralement entre 0,4 et 0,8 mg/kg de poids corporel du patient. La durée du traitement d'attaque est de l'ordre de 2 à 3 semaines à 5 semaines.

Les traitements d'entretien pour éviter les rechutes comprennent une administration de la dose utile une à deux fois par semaine pendant une ou deux semaines.

Il est à noter que l'activité du médicament selon l'invention est obtenue sans aucune irradiation des patients par rayonnements U.V.A.

## Revendications

1. Utilisation du 5-méthoxy-psoralène pour la préparation d'un médicament destiné au traitement spécifique de l'état dépressif et administrable a une dose journalière le soir au coucher de 0.2 milligramme à 1 milligramme par Kilo de poids corporel du patient.

2. Utilisation du 5-méthoxy-psoralène pour préparation d'un médicament antidépresseur selon la revendication 1, caractérisé en ce qu'il est destiné a être administré par voie orale sous forme de comprimés ou de gélules dosées à 20 mg de 5-méthoxy-psoralène.

3. Utilisation du 5-méthoxy-psoralène pour la préparation d'un médicament antidépresseur selon la revendication 1, caractérisé en ce qu'il est destiné a être administré par voie rectale sous forme de suppositoires dosés à 10 mg de 5-méthoxy-psoralène.

## Claims

1. The use of 5-methoxy-psoralene for the preparation of a medicament intended for the specific treatment of depression and able to be administered at a daily dose at night on going to bed of 0.2 milligram to 1 milligram per kilo body weight of the patient.

2. The use of 5-methoxy-psoralene for the preparation of an antidepressant medicament according to Claim 1, characterised in that it is intended to be administered orally in the form of tablets or capsules at a dosage of 20 mg 5-methoxy-psoralene.

3. The use of 5-methoxy-psoralene for the preparation of an antidepressant medicament according to Claim 1, characterised in that it is intended to be administered rectally in the form of suppositories at a dosage of 10 mg of 5-methoxy-psoralene.

## Patentansprüche

1. Verwendung von 5-Methoxy-psoralen zur Herstellung eines Arzneimittels, welches zur Behandlung spezifischer depressiver Zustände bestimmt ist und das bei einer Tagesdosis am Abend beim Schlafengehen von 0,2 mg bis 1 mg/kg Körpergewicht des Patienten verabreichbar ist.

2. Verwendung von 5-Methoxy-psoralen zur Herstellung eines Antidepressivum-Arzneimittels gemäß Anspruch 1, dadurch gekennzeichnet, daß es dazu bestimmt ist, auf oralem Wege in Form von Tabletten oder Gelkapseln mit einer Dosierung von 20 mg 5-Methoxy-psoralen verabreicht zu werden.

3. Verwendung von 5-Methoxy-psoralen zur Herstellung eines Antidepressivum-Arzneimittels gemäß Anspruch 1, dadurch gekennzeichnet, daß es dazu bestimmt ist, auf rektalem Wege in Form von Zäpfchen mit einer Dosierung von 10 mg 5-Methoxy-psoralen verabreicht zu werden.